# EUROPEAN PATENT SPECIFICATION

(11) **EP 1 047 690 B1**
(45) Date of publication and mention of the grant of the patent: **24.03.2004**
(21) Application number: 98964167.5
(22) Date of filing: 18.12.1998
(51) Int. Cl.: C07D 401/12, C07D 403/12, C07D 417/12, C07D 487/04, C07D 455/02, A61K 31/44, A61K 31/40, A61K 31/425, A61K 31/50, A61K 31/505, A61K 31/47

(54) **HETEROCYCLIC ETHER AND THIOETHER COMPOUNDS USEFUL IN CONTROLLING CHEMICAL SYNAPTIC TRANSMISSION**
HETEROZYKLISCHE ETHER- UND THIOETHER-VERBINDUNGEN, VERWENDBAR ZUR STEUERUNG VON CHEMISCHER SYNAPTISCHER TRANSMISSION
COMPOSES ETHER ET THIOETHER HETEROCYCLIQUES UTILISES DANS LA REGULATION DE LA TRANSMISSION SYNAPTIQUE CHIMIQUE

(30) Priority: 19.12.1997 US 994812
(43) Date of publication of application: 02.11.2000
(73) Proprietor: Abbott Laboratories, Abbott Park, Illinois 60064-3500 (US)
(72) Inventor: HOLLADAY, Mark, W., Tucson, AZ 85750 (US); ABREO, Melwyn, A., Imperial Beach, CA 91932 (US); GUNN, David, E., Hamden, CT 06517 (US); LIN, Nan-Horng, Mundelein, IL 60060 (US); GARVEY, David, S., Dover, MA 02030 (US); RYTHER, Keith, Round Lake Park, IL 60073 (US); LEBOLD, Suzanne, A., Chicago, IL 60614 (US); ELLIOTT, Richard, L., Easy Lyme, CT 06333 (US); HE, Yun, San Diego, CA 92122 (US); WASIAK, James, T., Waterford, WI 53185 (US); BAI, Hao, Grayslake, IL 60030 (US); DART, Michael, J., Highland Park, IL 60035 (US); EHRLICH, Paul, P., Guilford, CT 06437 (US); LI, Yihong, Grayslake, IL 60030 (US); KINCAID, John, F., Evanston, IL 60202 (US); SCHKERYANTZ, Jeffrey, M., Winthrop Harbor, IL 60096 (US); LYNCH, John, K., Kenosha, WI 53142 (US)
(74) Representative: Modiano, Guido, Dr.-Ing.
(86) International application number: PCT/US1998/027144
(87) International publication number: WO 1999/032480

(56) References cited:
- WO-A-94/08992
- WO-A-96/40682
- WO-A-97/20819
- US-A- 5 629 325
- HOLLADAY M W ET AL: "Structure-activity studies related to ABT-594, a potent nonopioid analgesic agent: effect of pyridine and azetidine ring substitutions on nicotinic acetylcholine receptor binding affinity and analgesic activity in mice" BIOORGANIC & MEDICINAL CHEMISTRY LETTERS, vol. 8, no. 19, 6 October 1998, page 2797-2802 XP004139623
- ABREO M A ET AL: "NOVEL 3-PYRIDYL ETHERS WITH SUBNANOMOLAR AFFINITY FOR CENTRAL NEURONAL NICOTINIC ACETYLCHOLINE RECEPTORS" JOURNAL OF MEDICINAL CHEMISTRY, vol. 39, no. 4, 1996, pages 817-825, XP002061887

## Description

This invention relates to heterocyclic ether and thioether compounds which control chemical synaptic transmission; to therapeutically-effective pharmaceutical compositions of these compounds; and to the use of said compositions to selectively control synaptic transmission.

Compounds that selectively control chemical synaptic transmission offer therapeutic utility in treating disorders that are associated with dysfunctions in synaptic transmission. This utility may arise from controlling either pre-synaptic or post-synaptic chemical transmission. The control of synaptic chemical transmission is, in turn, a direct result of a modulation of the excitability of the synaptic membrane. Presynaptic control of membrane excitability results from the direct effect an active compound has upon the organelles and enzymes present in the nerve terminal for synthesizing, storing, and releasing the neurotransmitter, as well as the process for active re-uptake. Postsynaptic control of membrane excitability results from the influence an active compound has upon the cytoplasmic organelles that respond to neurotransmitter action.

An explanation of the processes involved in chemical synaptic transmission will help to illustrate more fully the potential applications of the invention. (For a fuller explanation of chemical synaptic transmission refer to Hoffman *et al.*, "Neurotransmission: The autonomic and somatic motor nervous systems." In: Goodman and Gilman's, The Pharmacological Basis of therapeutics, 9th ed., J.G. Hardman, L.E. Limbird, P.B. Molinoff. R.W. Ruddon, and A. Goodman Gilman, eds.. Pergamon Press, New York, 1996, pp. 105-139).

Typically, chemical synaptic transmission begins with a stimulus that depolarizes the transmembrane potential of the synaptic junction above the threshold that elicits an all-or-none action potential in a nerve axon. The action potential propagates to the nerve terminal where *ion fluxes* activate a mobilization process leading to neurotransmitter secretion and "transmission" to the postsynaptic cell. Those cells which receive communication from the central and peripheral nervous systems in the form of neurotransmitters are referred to as "excitable cells." Excitable cells are cells such as nerves, smooth muscle cells, cardiac cells and glands. The effect of a neurotransmitter upon an excitable cell may be to cause either an excitatory or an inhibitory postsynaptic potential (EPSP or IPSP, respectively) depending upon the nature of the postsynaptic receptor for the particular neurotransmitter and the extent to which other neurotransmitters are present. Whether a particular neurotransmitter causes excitation or inhibition depends principally on the ionic channels that are opened in the postsynaptic membrane (i.e., in the excitable cell).

EPSPs typically result from a local depolarization of the membrane due to a generalized increased permeability to cations (notably Na⁺ and K⁺), whereas IPSPs are the result of stabilization or hyperpolarization of the membrane excitability due to a increase in permeability to primarily smaller ions (including K⁺ and Cl⁻). For example, the neurotransmitter acetylcholine excites at skeletal muscle junctions by opening permeability channels for Na⁺ and K⁺. At other synapses, such as cardiac cells, acetylcholine can be inhibitory, primarily resulting from an increase in K⁺ conductance.

The biological effects of the compounds of the present invention result from modulation of a particular subtype of acetylcholine receptor. It is, therefore, important to understand the differences between two receptor subtypes. The two distinct subfamilies of acetylcholine receptors are defined as nicotinic acetylcholine receptors and muscarinic acetylcholine receptors. (See Goodman and Gilman's, The Pharmacological Basis of Therapeutics, *op. cit.).*

The responses of these receptor subtypes are mediated by two entirely different classes of second messenger systems. When the nicotinic acetylcholine receptor is activated, the response is an increased flux of specific extracellular ions (e.g. Na⁺, K⁺ and Ca⁺⁺) through the neuronal membrane. In contrast, muscarinic acetylcholine receptor activation leads to changes in intracellular systems that contain complex molecules such as G-proteins and inositol phosphates. Thus, the biological consequences of nicotinic acetylcholine receptor activation are distinct from those of muscarinic receptor activation. In an analogous manner, inhibition of nicotinic acetylcholine receptors results in still other biological effects, which are distinct and different from those arising from muscarinic receptor inhibition.

As indicated above, the two principal sites to which drug compounds that affect chemical synaptic transmission may be directed are the presynaptic membrane. Actions of drugs directed to the presynaptic site may be mediated through presynaptic receptors that respond to the neurotransmitter which the same secreting structure has released (i.e., through an autoreceptor), or through a presynaptic receptor that responds to another neurotransmitter (i.e., through a heteroreceptor). Actions of drugs directed to the postsynaptic membrane mimic the action of the endogenous neurotransmitter or inhibit the interaction of the endogenous neurotransmitter with a postsynaptic receptor.

Classic examples of drugs that modulate postsynaptic membrane excitability are the neuromuscular blocking agents which interact with nicotinic acetylcholine-gated channel receptors on skeletal muscle, for example, competitive (stabilizing) agents, such as curare, or depolarizing agents, such as succinylcholine.

In the central nervous system, postsynaptic cells can have many neurotransmitters impinging upon them. This makes it difficult to know the precise net balance of chemical synaptic transmission required to control a given cell. Nonetheless, by designing compounds that selectively affect only one pre- or postsynaptic receptor, it is possible to modulate the net balance of all the other inputs. Obviously, the more that is understood about chemical synaptic transmission in CNS disorders. the easier it would be to design drugs to treat such disorders.

Knowing how specific neurotransmitters act in the CNS allows one to speculate about the disorders that may be treatable with certain CNS-active drugs. For example, dopamine is widely recognized as an important neurotransmitter in the central nervous systems in humans and animals. Many aspects of the pharmacology of dopamine have been reviewed by Roth and Elsworth, "Biochemical Pharmacology of Midbrain Dopamine Neurons". In: Psychopharmacology: The Fourth Generation of Progress, F.E. Bloom and D.J. Kupfer. Eds., Raven Press, NY, **1995**, pp 227-243). Patients with Parkinson's disease have a primary loss of dopamine containing neurons of the nigrostriatal pathway, which results in profound loss of motor control. Therapeutic strategies to replace the dopamine deficiency with dopamine mimetics. as well as administering pharmacologic agents that modify dopamine release and other neurotransmitters have been found to have therapeutic benefit ("Parkinson's Disease", In: Psychopharmacology: The Fourth Generation of Progress, *op. cit.,* pp 1479-1484).

New and selective neurotransmitter controlling agents are still being sought, in the hope that one or more will be useful in important, but as yet poorly controlled, disease states or behavior models. For example, dementia, such as is seen with Alzheimer's disease or Parkinsonism, remains largely untreatable. Symptoms of chronic alcoholism and nicotine withdrawal involve aspects of the central nervous system, as does the behavioral disorder Attention-Deficit Disorder (ADD). Specific agents for treatment of these and related disorders are few in number or non-existent.

A more complete discussion of the possible utility as CNS-active agents of compounds with activity as cholinergic ligands selective for neuronal nicotinic receptors, *(i.e.,* for controlling chemical synaptic transmission) may be found in U.S. Patent 5,472,958, to Gunn et al., issued Dec. 5, 1995.

Existing acetylcholine agonists are therapeutically suboptimal in treating the conditions discussed above. For example, such compounds have unfavorable pharmacokinetics (e.g., arecoline and nicotine), poor potency and lack of selectivity (e.g., nicotine), poor CNS penetration (e.g., carbachol) or poor oral bioavailability (e.g., nicotine). In addition, other agents have many unwanted central agonist actions, including hypothermia, hypolocomotion and tremor and peripheral side effects, including miosis, lachrymation, defecation and tachycardia (Benowitz *et al.,* in: Nicotine Psychopharmacology, S. Wonnacott, M.A.H. Russell, & I.P. Stolerman, eds., Oxford University Press, Oxford, **1990**, pp. 112-157: and M. Davidson, *et al.,* in Current Research in Alzheimer Therapy, E. Giacobini and R. Becker, ed.; Taylor & Francis: New York, **1988**; pp 333-336).

Williams et al. reports the use of cholinergic channel modulators to treat Parkinson's and Alzheimer's Diseases. M. Williams et al., "Beyond the Tobacco Debate: Dissecting Out the Therapeutic Potential of Nicotine," Exp. Opin. Invest. Drugs 5, pp. 1035-1045 (1996). Salin-Pascual et al. reports short-term improvement of non-smoking patients suffering from depression by treatment with nicotine patches. R. J.Salin-Pascual et al.. "Antidepressant Effect of Transdermal Nicotine Patches in Non-Smoking Patients with Major Depression," J. Clin. Psychiatry, v. 57 pp. 387-389 (1996).

Various heterocyclic 2-pyrrolidinyloxy-substituted compounds with analgesic and hypotensive activities have been disclosed by Scheffler *et al.* (U.S. Patent 4,643,995) and Tomioka *et al.* (*Chem. Pharm. Bull,* 38:2133-5, **1990**).

Certain other 2-pyridyloxy-substituted compounds are disclosed *inter alia* by Engel *et al.* in U.S. Patent 4.946.836 as having analgesic activity.

Various other compounds having a pyrrolidine or azetidine moiety substituted at the 3-position have also been disclosed (*cf.* U.S. Patents 4,592,866 to A.D. Cale; 4,705,853 to A.D. Cale; 4,956,359 to Taylor *et al.;*and 5.037,841 to Schoehe *et al.* and European patent application EP296560A2, to Sugimoto *et al.*).

Certain nicotine-related compounds having utility in enhancing cognitive function have been reported by Lin in U.S. Patent 5,278,176, issued Jan. 11, 1994. Also, 2-(nitro)phenoxy compounds with similar function have been reported by Gunn *et al.,* U.S. Patent 5,472,958, issued Dec. 5, 1995.

Certain 3-pyridyloxymethyl heterocyclic ether compounds useful in controlling chemical synaptic transmission have been described by Lin et al. in U.S. Patent 5,629,325, issued May 13, 1997.

In the PCT Patent Application WO94/08992 of Abreo *et al.,* published April 28, 1994, are disclosed, *inter alia,* various 3-pyridyloxy-heterocyclic compounds that are either unsubstituted or mono-substituted on the pyridine ring with groups such as Br, Cl, F, hydroxyl, C₁-C₃-alkyl or C₁-C₃-alkoxy, such compounds also described as having utility in enhancing cognitive function.

### Summary of the Invention

It has been found. in accordance with the present invention. that a class of heterocyclic ether and thioether compounds are selective and potent neuronal nicotinic cholinergic compounds useful in controlling synaptic transmission.

In its principal aspect, the present invention provides compounds, as well as pharmaceutically acceptable salts and prodrugs thereof, selected from the group consisting of 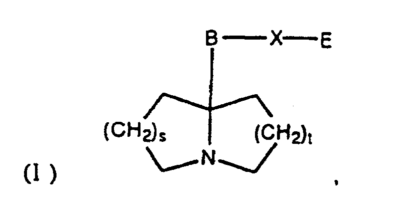 wherein
B is 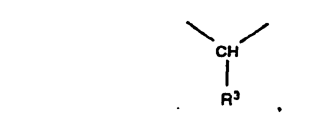 wherein R³ is H or C₁-C₆-alkyl;
X is selected from the group consisting of oxygen and sulfur; and
E is 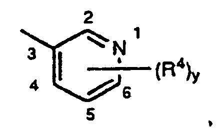 wherein
y is an integer selected from 2 and 3, with the requirements that
(a) when y=2,
   R⁴ is selected from the group having substituents at the 2,4-, 2,5-, 2,6-, 4,5-, 4,6- and 5,6- positions of the pyridine ring wherein
   a 2-position substituent is selected from the group consisting of
   (i) -Br,
   (ii) -Cl,
   (iii) -F,
   (iv) -OH,
   (v) -NH₂,
   (vi) -C₁-C₄-alkyl, and
   (vii) -C₁-C₃-allcoxy; and
   a substituent at the 4-, 5- and 6-position of the pyridine ring is selected from the group consisting of
   (i) -Br,
   (ii) -Cl,
   (iii) -F,
   (iv) -OH,
   (v) -C₁-C₄-alkyl,
   (vi) -CN,
   (vii) -CH₂F,
   (viii) -CHF₂,
   (ix) -CF₃,
   (x) -NO₂,
   (xi) -CH₂OH,
   (xii) -CH₂CN,
   (xiii) -C₁-C₃-alkoxy,
   (xiv) -NH₂,
   (xv) -NH-CHO,
   (xvi) -NH-C(O)-(C₁-C₃-alkyl),
   (xvii) -N(C₁-C₃-alkyl)-C(O)-(C₁-C₃-alkyl),
   (xviii) -NH-(C₁-C₃-alkyl),
   (xix) -NH-CH₂-phenyl,
   (xx) -N(C₁-C₃-alkyl)₂,
   (xxi) -C(O)-OH,
   (xxii) -C(O)-O-C₁-C₃-alkyl,
   (xxiii) -C(O)-NH₂,
   (xxiv) -C(O)-NH-C₁-C₃-alkyl,
   (xxv) -C(O)-NH-CH₂-phenyl, and
   (xxvi) -O-C(O)-(C₁-C₃-alkyl); and
   with the requirement that when there is no 2-position substitutent, then one R⁴ substituent must be selected from the group consisting of:
   (i) -Br,
   (ii) -Cl,
   (iii) -F,
   (iv) -CN,
   (v) -CH₂OH,
   (vi) -C₁-C₄-alkyl, and
(b) when y=3,
   R⁴ is selected from the group having substituents at the 2,4,5-, 2,4,6-, 2.5.6- and 4,5,6- positions of the pyridine ring wherein
   a 2-position substituent is selected from the group consisting of
   (i) -Br,
   (ii) -Cl,
   (ii) -F,
   (iv) -OH,
   (v) -C₁-C₄-alkyl, and
   (vi) -C₁-C₃ alkoxy; and
   a substituent at the 4-, 5- and 6-position of the pyridine ring is selected from the group consisting of
   (i) -Br,
   (ii) -Cl,
   (iii) -F,
   (iv) -OH,
   (v) -C₁-C₄-alkyl,
   (vi) -CN,
   (vii) -CH₂F,
   (viii) -CHF₂,
   (ix) -CF₃,
   (x) -NO₂,
   (xi) -CH₂OH,
   (xii) -CH₂CN,
   (xiii) -C₁-C₃-alkoxy,
   (xiv) -NH₂,
   (xv) -NH-CHO,
   (xvi) -NH-C(O)-(C₁-C₃-alkyl),
   (xvii) -N(C₁-C₃-alkyl)-C(O)-(C₁-C₃-alkyl),
   (xviii) -NH-(C₁-C₃-alkyl),
   (xix) -NH-CH₂-phenyl,
   (xx) -N(C₁-C₃-alkyl)₂,
   (xxi) -C(O)-OH,
   (xxii)-C(O)-O-C₁-C₃-alkyl,
   (xxiii) -C(O)-NH₂,
   (xxiv) -C(O)-NH-C₁-C₃-alkyl,
   (xxv) -C(O)-NH-CH2-phenyl, and
   (xxvi) -O-C(O)-(C₁-C₃-alkyl): and
   with the requirement that when there is no 2-position substitutent, then two R⁴ substituents must be selected from the group consisting of
   (i) -Br,
   (ii) -Cl,
   (iii) -F,
   (iv) -CN,
   (v) -CH₂OH,
   (vi) -C₁-C₄-alkyl
   and
s and t are integers independently selected from the group consisting of 0, 1 and 2, with the requirement that both s and t may not concurrently be 0;
and 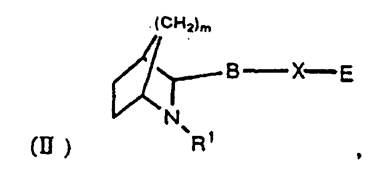 wherein m is selected from the group consisting of 1 and 2, and
B, X, E are as defined above and
R¹ is selected from the group consisting of H, allyl and C₁-C₆-alkyl.

The invention also provides a pharmaceutical composition for controlling synaptic transmission. comprising a pharmaceutically-acceptable carrier and a therapeutically-effective amount of a compound selected from the group having the formulas (I) and (II) described previously.

The invention also provides the use of a compound selected from the group having the formulas (I) and (II) described previously for manufacturing a medicament for selectively controlling synaptic transmission by administration to a human or veterinary patient in need of such treatment of a therapeutically-effective amount.

The invention further provides the use of a compound that selectively controls synaptic transmission selected from the group having the formulas (I) and (II) described previously for manufacturing a medicament for treating dementias, attention-deficit disorder, anxiety associated with cognitive impairment or substance abuse withdrawal characterized by decreased cholinergic function by administration to a human or veterinary patient in need of such treatment of a therapeutically-effective amount.

### Detailed Description of the Invention

A preferred embodiment of the invention is a compound of formula (I) wherein X is oxygen.

A further preferred embodiment of the invention is a compound of formula (II) wherein X is oxygen.

Representative compounds of the invention are those selected from the group consisting of:
(1S,4R)-3-(S)-(5,6-dichloro-3-pyridyloxymethyl)-N-methyl-2-azabicyclo[2.2.1]heptane;
(1S,4R)-3-(S)-(5-bromo-6-chloro-3-pyridyloxymethyl)-N-methyl-2-azabicyclo [2.2.1] heptane;
(1S,4R)-3-(S)-(5-amino-3-pyridyloxymethyl)-N-methyl-2-azabicyclo[2.2.1]heptane;

### Definitions

The terms "C₁-C₃-alkyl," "C₁-C₄-alkyl," or "C₁-C₆-alkyl" as used herein refer to saturated, straight- or branched-chain hydrocarbon radicals containing between one and three or one and six carbon atoms, respectively. Examples of C₁-C₄-alkyl radicals include, but are not limited to, methyl, ethyl, propyl, isopropyl, *n*-butyl and t-butyl. Examples of C₁-C₃ alkyl radicals include methyl, ethyl, propyl and isopropyl, examples of C₁-C₆-alkyl radicals include, but are not limited to, methyl, ethyl, propyl, isopropyl, *n*-butyl, *t*-butyl, neopentyl, n-hexyl.

The term "C₁-C₃-alkoxy" as used herein refers to an C₁-C₃-alkyl group, as previously defined, attached to the parent molecular moiety through an oxygen atom. Examples of C₁-C₃-alkoxy, but are not limited to, methoxy, ethoxy, propoxy and isopropoxy.

One or more asymmetric centers may exist in the compounds of the present invention. Except where otherwise noted, the present invention contemplates the various stereoisomers and mixtures thereof.

As used herein, the term "pharmaceutically acceptable salt" refers to those salts which are, within the scope of sound medical judgment, suitable for use in contact with the tissues of humans and lower animals without undue toxicity, irritation, allergic response and the like, and are commensurate with a reasonable benefit/risk ratio. Pharmaceutically acceptable salts are well known in the art. For example, S. M. Berge, *et al.* describe pharmaceutically acceptable salts in detail in J. Pharmaceutical Sciences, 66: 1-19 (1977). The salts can be prepared *in situ* during the final isolation and purification of the compounds of the invention, or separately by reacting the free base function with a suitable organic acid. Examples of pharmaceutically acceptable, nontoxic acid addition salts are salts of an amino group formed with inorganic acids such as hydrochloric acid, hydrobromic acid, phosphoric acid, sulfuric acid and perchloric acid or with organic acids such as acetic acid, oxalic acid, maleic acid, tartaric acid, citric acid, succinic acid or malonic acid or by using other methods used in the art such as ion exchange. Other pharmaceutically acceptable salts include adipate, alginate, ascorbate, aspartate, benzenesulfonate, benzoate, bisulfate, borate, butyrate, camphorate, camphorsulfonate, citrate, cyclopentanepropionate, digluconate, dodecylsulfate, ethanesulfonate, formate, fumarate, glucoheptonate, glycerophosphate, gluconate, hemisulfate, heptanoate, hexanoate, hydroiodide, 2-hydroxy-ethanesulfonate, lactobionate, lactate, laurate, lauryl sulfate, malate, maleate, malonate, methanesulfonate, 2-naphthalenesulfonate, nicotinate, nitrate, oleate, oxalate, palmitate, pamoate, pectinate, persulfate, 3-phenylpropionate, phosphate, picrate, pivalate, propionate, stearate, succinate, sulfate, tartrate, thiocyanate, p-toluenesulfonate, undecanoate, valerate salts, and the like. Representative alkali or alkaline earth metal salts include sodium, lithium, potassium, calcium, magnesium, and the like. Further pharmaceutically acceptable salts include, when appropriate, nontoxic ammonium, quaternary ammonium, and amine cations formed using counterions such as halide, hydroxide, carboxylate, sulfate, phosphate, nitrate, lower alkyl sulfonate and aryl sulfonate.

The term "prodrug" refers to compounds that are rapidly transformed *in vivo* to yield the parent compounds of Formula (I), as for example. by hydrolysis in blood. T. Higuchi and V. Stella provide a thorough discussion of the prodrug concept in Prodrugs as Novel Delivery Systems, Vol. 14 of the A.C.S. Symposium Series, American Chemical Society (1975). Examples of esters useful as prodrugs for compounds containing carboxyl groups may be found on pages 14-21 of Bioreversible Carriers in Drug Design: Theory and Application, edited by E.B. Roche. Pergamon Press (1987). Specific prodrug moieties that are useful include, for example, 1-acetoxy-(1-methyl)ethoxycarbonyl. acetyl. 2-(hydroxymethyl)benzoyl, 2-methylphenoxycarbonyl, 2-oxo-tetrahydrofuran-4-(R)-carboxoyl, 2-oxo-tetrahydrofuran-4-(S)-carboxoyl, 3-oxocyclohexenyl, 4-(diethylaminomethyl)benzoyl, 4-(methoxycarbonyl)phenoxycarbonyl, 4-chlorophenoxycarbonyl. 4-fluorophenoxycarbonyl, 4-methoxyphenoxycarbonyl, 4-methylphenoxycarbonyl, 4-nitrophenoxycarbonyl, (5-methyl-2-oxo- 1, 3-dioxol-4-en-4-yl)methoxycarbonyl, (5-methyl-2-oxo- 1,3-dioxol-4-en-4-yl)methoxycarbonyl, 2, 2-bis(ethoxycarbonyl)ethenyl, 2, 5-dihydro-2-oxo-furan-4-yl, 2, 6-dimethylphenoxycarbonyl, 5,5-dimethyl-3-oxocyclohexenyl, BOC, D-alanyl, D-phenylalanyl, ethoxycarbonyl, L-alanyl, L-phenylalanyl, monomethyl phthalyl, N-(2-hydroxybenzoyl)aminomethyl, N-acetyl-D-alanyl, N-aceryl-D-phenylalanyl, N-acetyl-L-alanyl, N-acetyl-L-phenylalanyl, N-BOC-D-alanyl, N-BOC-D-phenylalanyl, N-BOC-L-alanyl, N-BOC-L-phenylalanyl, N-phthalimidylmethyl, (pyrrolidin-1-yl)carbonyl, N-succinimidylmethyl, phenoxycarbonyl, (pyrrolidin-1-yl)carbonyl, and S-(phenylmethyl)cysteinoyl.

The term "prodrug ester group" refers to any of several ester-forming groups that are hydrolyzed under physiological conditions. Examples of prodrug ester groups include pivaloyloxymethyl, acetoxymethyl, phthalidyl, indanyl and methoxymethyl, as well as other such groups known in the art.

As used herein, the term "pharmaceutically acceptable ester" refers to esters which hydrolyze *in vivo* and include those that break down readily in the human body to leave the parent compound or a salt thereof. Suitable ester groups include, for example, those derived from pharmaceutically acceptable aliphatic carboxylic acids, particularly alkanoic, alkenoic, cycloalkanoic and alkanedioic acids, in which each alkyl or alkenyl moiety advantageously has not more than 6 carbon atoms. Examples of particular esters includes formates, acetates, propionates, butyrates, acrylates and ethylsuccinates.

### Abbreviations

Abbreviations which have been used in the descriptions of the scheme and the examples that follow are: BOC for t-butyloxycarbonyl; Et₂O for diethyl ether; EtOAc for ethyl acetate; DEAD for diethylazodicarboxylate: DMAP for 4-dimethylaminopyridine: DMF for dimethyl formamide; DPPA for diphenylphosphoryl azide; EDC for 1-(3-dimethylaminopropyl)-3-ethylcarbodiimide HCl; EtOAc for ethyl acetate; MeOH for methanol; NaN(TMS)₂ for sodium bis(trimethylsilyl)amide; NMMO for N-methylmorpholine N-oxide; Ph for phenyl; TEA for triethylamine; TFA for trifluoroacetic acid; THF for tetrahydrofuran; TPP for triphenylphosphine.

### Pharmaceutical Compositions

The pharmaceutical compositions of the present invention comprise a therapeutically effective amount of a compound of the present invention formulated together with one or more pharmaceutically acceptable carriers. As used herein, the term "pharmaceutically acceptable carrier" means a non-toxic, inert solid, semi-solid or liquid filler, diluent, encapsulating material or formulation auxiliary of any type. Some examples of materials which can serve as pharmaceutically acceptable carriers are sugars such as lactose, glucose and sucrose; starches such as corn starch and potato starch; cellulose and its derivatives such as sodium carboxymethyl cellulose, ethyl cellulose and cellulose acetate; powdered tragacanth; malt; gelatin; talc; excipients such as cocoa butter and suppository waxes; oils such as peanut oil, cottonseed oil; safflower oil; sesame oil; olive oil; corn oil and soybean oil; glycols such a propylene glycol; esters such as ethyl oleate and ethyl laurate; agar; buffering agents such as magnesium hydroxide and aluminum hydroxide; alginic acid; pyrogen-free water; isotonic saline; Ringer's solution; ethyl alcohol, and phosphate buffer solutions, as well as other non-toxic compatible lubricants such as sodium lauryl sulfate and magnesium stearate. as well as coloring agents, releasing agents, coating agents, sweetening, flavoring and perfuming agents, preservatives and antioxidants can also be present in the composition, according to the judgment of the formulator. The pharmaceutical compositions of this invention can be administered to humans and other animals orally, rectally, parenterally, intracisternally, intravaginally, intraperitoneally, topically (as by powders, ointments, or drops), bucally, or as an oral or nasal spray.

Liquid dosage forms for oral administration include pharmaceutically acceptable emulsions, microemulsions, solutions, suspensions, syrups and elixirs. In addition to the active compounds, the liquid dosage forms may contain inert diluents commonly used in the art such as, for example, water or other solvents, solubilizing agents and emulsifiers such as ethyl alcohol, isopropyl alcohol, ethyl carbonate, ethyl acetate, benzyl alcohol, benzyl benzoate, propylene glycol, 1,3-butylene glycol, dimethylformamide, oils (in particular, cottonseed, groundnut, corn, germ, olive. castor, and sesame oils), glycerol, tetrahydrofurfuryl alcohol, polyethylene glycols and fatty acid esters of sorbitan, and mixtures thereof. Besides inert diluents, the oral compositions can also include adjuvants such as wetting agents. emulsifying and suspending agents, sweetening, flavoring, and perfuming agents.

Injectable preparations, for example, sterile injectable aqueous or oleaginous suspensions may be formulated according to the known art using suitable dispersing or wetting agents and suspending agents. The sterile injectable preparation may also be a sterile injectable solution, suspension or emulsion in a nontoxic parenterally acceptable diluent or solvent, for example, as a solution in 1,3-butanediol. Among the acceptable vehicles and solvents that may be employed are water, Ringer's solution, U.S.P. and isotonic sodium chloride solution. In addition, sterile, fixed oils are conventionally employed as a solvent or suspending medium. For this purpose any bland fixed oil can be employed including synthetic mono- or diglycerides. In addition, fatty acids such as oleic acid are used in the preparation of injectables.

The injectable formulations can be sterilized, for example, by filtration through a bacterial-retaining filter, or by incorporating sterilizing agents in the form of sterile solid compositions which can be dissolved or dispersed in sterile water or other sterile injectable medium prior to use.

In order to prolong the effect of a drug, it is often desirable to slow the absorption of the drug from subcutaneous or intramuscular injection. This may be accomplished by the use of a liquid suspension of crystalline or amorphous material with poor water solubility. The rate of absorption of the drug then depends upon its rate of dissolution which, in turn, may depend upon crystal size and crystalline form. Alternatively, delayed absorption of a parenterally administered drug form is accomplished by dissolving or suspending the drug in an oil vehicle. Injectable depot forms are made by forming microencapsule matrices of the drug in biodegradable polymers such as polylactide-polyglycolide. Depending upon the ratio of drug to polymer and the nature of the particular polymer employed, the rate of drug release can be controlled. Examples of other biodegradable polymers include poly(orthoesters) and poly(anhydrides) Depot injectable formulations are also prepared by entrapping the drug in liposomes or microemulsions which are compatible with body tissues.

Compositions for rectal or vaginal administration are preferably suppositories which can be prepared by mixing the compounds of this invention with suitable non-irritating excipients or carriers such as cocoa butter, polyethylene glycol or a suppository wax which are solid at ambient temperature but liquid at body temperature and therefore melt in the rectum or vaginal cavity and release the active compound.

Solid dosage forms for oral administration include capsules, tablets, pills, powders, and granules. In such solid dosage forms, the active compound is mixed with at least one inert, pharmaceutically acceptable excipient or carrier such as sodium citrate or dicalcium phosphate and/or a) fillers or extenders such as starches, lactose, sucrose, glucose, mannitol, and silicic acid, b) binders such as, for example, carboxymethylcellulose, alginates, gelatin. polyvinylpyrrolidinone, sucrose, and acacia, c) humectants such as glycerol, d) disintegrating agents such as agar-agar, calcium carbonate, potato or tapioca starch, alginic acid, certain silicates, and sodium carbonate, e) solution retarding agents such as paraffin, f) absorption accelerators such as quaternary ammonium compounds, g) wetting agents such as, for example, cetyl alcohol and glycerol monostearate, h) absorbents such as kaolin and bentonite clay, and i) lubricants such as talc, calcium stearate, magnesium stearate, solid polyethylene glycols, sodium lauryl sulfate, and mixtures thereof. In the case of capsules, tablets and pills, the dosage form may also comprise buffering agents.

Solid compositions of a similar type may also be employed as fillers in soft and hard-filled gelatin capsules using such excipients as lactose or milk sugar as well as high molecular weight polyethylene glycols and the like.

The solid dosage forms of tablets, dragees, capsules, pills, and granules can be prepared with coatings and shells such as enteric coatings and other coatings well known in the pharmaceutical formulating art. They may optionally contain opacifying agents and can also be of a composition that they release the active ingredient(s) only, or preferentially, in a certain part of the intestinal tract, optionally, in a delayed manner. Examples of embedding compositions which can be used include polymeric substances and waxes.

Solid compositions of a similar type may also be employed as fillers in soft and hard-filled gelatin capsules using such excipients as lactose or milk sugar as well as high molecular weight polyethylene glycols and the like.

The active compounds can also be in micro-encapsulated form with one or more excipients as noted above. The solid dosage forms of tablets, dragees, capsules, pills, and granules can be prepared with coatings and shells such as enteric coatings, release controlling coatings and other coatings well known in the pharmaceutical formulating art. In such solid dosage forms the active compound may be admixed with at least one inert diluent such as sucrose, lactose or starch. Such dosage forms may also comprise, as is normal practice, additional substances other than inert diluents, e.g., tableting lubricants and other tableting aids such a magnesium stearate and microcrystalline cellulose. In the case of capsules, tablets and pills, the dosage forms may also comprise buffering agents. They may optionally contain opacifying agents and can also be of a composition that they release the active ingredient(s) only, or preferentially, in a certain part of the intestinal tract, optionally, in a delayed manner. Examples of embedding compositions which can be used include polymeric substances and waxes.

Dosage forms for topical or transdermal administration of a compound of this invention include ointments, pastes, creams, lotions, gels, powders, solutions, sprays, inhalants or patches. The active component is admixed under sterile conditions with a pharmaceutically acceptable carrier and any needed preservatives or buffers as may be required. Ophthalmic formulation, ear drops, eye ointments, powders and solutions are also contemplated as being within the scope of this invention.

The ointments, pastes, creams and gels may contain, in addition to an active compound of this invention, excipients such as animal and vegetable fats, oils, waxes, paraffins. starch, tragacanth, cellulose derivatives, polyethylene glycols, silicones, bentonites, silicic acid, talc and zinc oxide, or mixtures thereof.

Powders and sprays can contain, in addition to the compounds of this invention, excipients such as lactose, talc, silicic acid, aluminum hydroxide, calcium silicates and polyamide powder, or mixtures of these substances. Sprays can additionally contain customary propellants such as chlorofluorohydrocarbons.

Transdermal patches have the added advantage of providing controlled delivery of a compound to the body. Such dosage forms can be made by dissolving or dispensing the compound in the proper medium. Absorption enhancers can also be used to increase the flux of the compound across the skin. The rate can be controlled by either providing a rate controlling membrane or by dispersing the compound in a polymer matrix or gel.

According to the methods of treatment of the present invention. disorders in synaptic transmission are treated or prevented in a patient such as a human or lower mammal by administering to the patient a therapeutically effective amount of a compound of the invention in such amounts and for such time as is necessary to achieve the desired result. By a "therapeutically effective amount" of a compound of the invention is meant a sufficient amount of the compound to treat disorders in synaptic transmission, at a reasonable benefit/risk ratio applicable to any medical treatment. It will be understood, however, that the total daily usage of the compounds and compositions of the present invention will be decided by the attending physician within the scope of sound medical judgment. The specific therapeutically effective dose level for any particular patient will depend upon a variety of factors including the disorder being treated and the severity of the disorder; the activity of the specific compound employed; the specific composition employed; the age, body weight, general health, sex and diet of the patient; the time of administration, route of administration, and rate of excretion of the specific compound employed; the duration of the treatment; drugs used in combinadon or coincidental with the specific compound employed; and like factors well known in the medical arts.

### Therapeutic Administration

The total daily dose of the compounds of this invention administered to a human or other mammal in single or in divided doses can be in amounts, for example, from 0.00 to 50 mg/kg body weight or more usually from 0.001 to 25 mg/kg body weight. Single dose compositions may contain such amounts or submultiples thereof to make up the daily dose. In general, treatment regimens according to the present invention comprise administration to a patient in need of such treatment from about 30 mg to about 1000 mg, and preferably about 80 mg, of the compound(s) of this invention per day in single or multiple doses.

### Synthetic Methods

The compounds and processes of the present invention will be better understood in connection with the following synthetic schemes which illustrate the methods by which the compounds of the invention may be prepared. The groups n, y, R¹, R³ and R⁴ are as defined above unless otherwise noted.

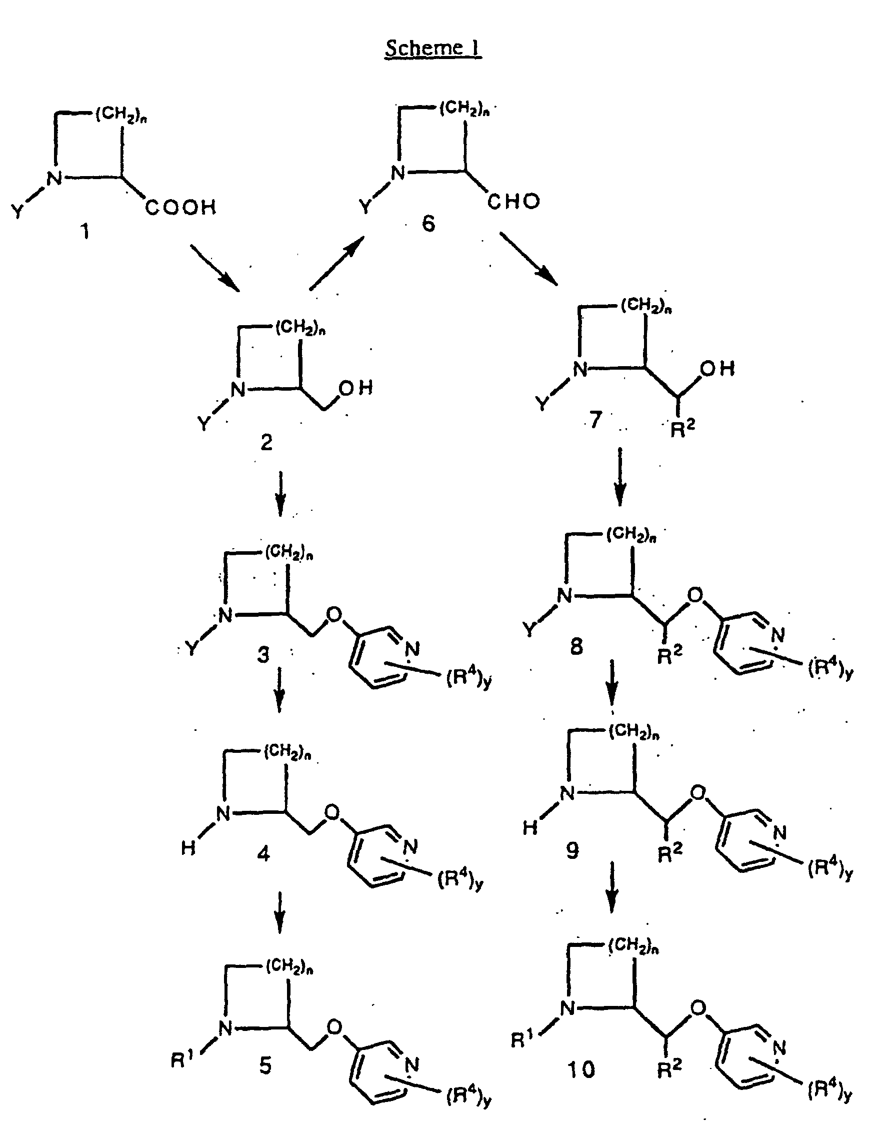

In accordance with reaction Scheme l, compounds 5 and 10 (not claimed) are prepared. A 2-carboxyl-substituted azacycloalkyl compound (1), wherein n is as described above, R³ is H and Y is a C₁-C₃-alkyl or a suitable protecting group, such as BOC or CBZ, for example, which may subsequently be removed and replaced with H, allyl, or C₁-C₃-alkyl, is converted to the hydroxymethyl compound of formula (2) with a suitable reducing agent, such as Red-Al®, borane/THF, borane/methyl sulfide or LiAlH₄, for example. Compound (2) is reacted with an appropriately substituted 3-hydroxypyridine compound, wherein R⁴ is as described above or is an appropriately protected R⁴ group (wherein the protecting group may be removed after the coupling reaction), in the presence of triphenylphosphine and DEAD such as described by O. Mitsunobu (*Synthesis.* **1981**: 1) to form the pyridine compound of formula (3). This compound is then treated with a reagent suitable for removing the N-protecting group, such as trifluoroacetic acid for BOC removal, HCl in ethanol for CBZ removal, HBr in acetic acid, or hydrogenolysis with hydrogen in the presence of a noble mecal catalyst, to form the unprotected compound (4).

The ring nitrogen may then be alkylated, for example, by treatment with alkyl halide in the presence of a base, formaldehyde in formic acid, or with an aldehyde and sodium cyanoborohydride in an alcohol solvent, such as methanol, ethanol or isopropanol, or by reaction with the appropriate alkylating reagent, such as, for example, allyl bromide, to give the desired compound (5), wherein R¹ is as described above.

Alternately, in place of the Mitsunobu reaction described above, it is possible to react the free hydroxyl group of compound (2) with a suitable reagent to convert it to a leaving group, such as tosylate or mesylate, for example, then reacting this compound with a suitable 3-pyridinol compound in the presence of base to prepare compounds (3). In addition, it is possible to react the free hydroxyl compound with a strong base to convert it to the alkoxide ion and react the alkoxide with a suitable 3-halopyridine compound to to prepare compounds (3). This reaction may be partiularly useful when other electron-withdrawing substituents are present on the pyridine ring.

Alternately, compound (2) may be oxidized using a suitable mild oxidizing agent such as DMSO/pyridine•SO₃, pyridinium chlorochromate or DMSO/oxalyl chloride, for example, to afford the aldehyde (6). The aldehyde is then reacted with a suitable organometallic nucleophile, for example, a Grignard reagent, to afford the alcohol (7), wherein R² is as described above. The alcohol is then reacted as described above with a 3-hydroxypyridine and carried through the same series of reactions as described above, starting with compound (2) and leading to compounds (3), (4) and (5), to give the compounds (8), (9) and (10), wherein R¹ is as described above.

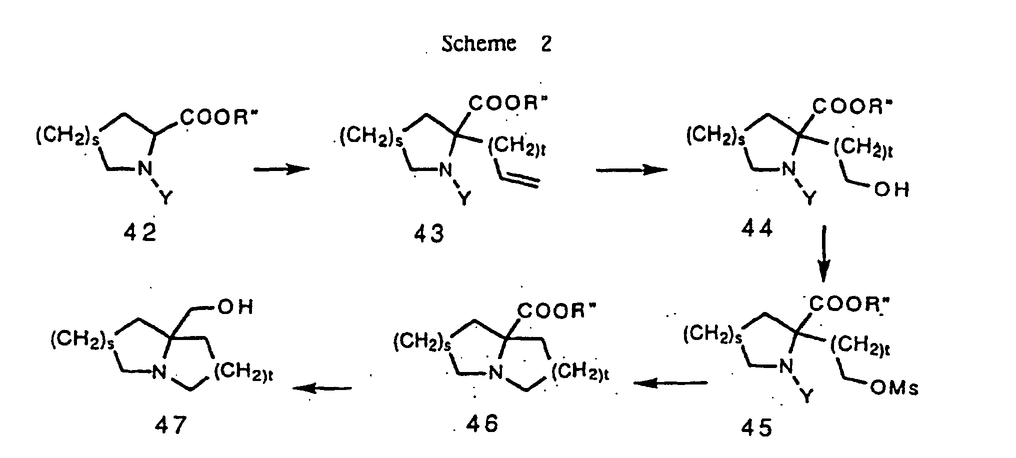

In accordance with Scheme 2, it is possible to prepare the precursors of compounds of formula (I). The compound (42), wherein s is 0, 1, or 2, as desired, R' is a suitable C₁-C₅-alkyl group, and Y is an N-protecting group, such as CBZ, is reacted with LDA, followed by either 3-bromo-1-propene or 4-bromo-1-butene, to give compound (43), wherein t is 1 or 2, respectively. In the instance wherein it is desired than be 1 or 2, compound (43) is reacted with BH₃ followed by oxidation with H₂O₂ to give the alcohol compound (44). In the alternate instance wherein it is desired that t be 0, compound (43) is first oxidized with NalO₄ and OsO₄, followed by reduction with NaBH₄, to give the compound (44) wherein t is 0. The alcohol (44), wherein t is 0, 1, or 2, is then reacted with methanesulfonyl chloride in a non-polar solvent and presence of a base, such as triethylamine, to give compound (45). By removal of protecting group Y from (45) under standard conditions, followed by treatment with base, such as K₂CO₃ or triethylamine, the ring closure is accomplished to afford compound (46). Reduction of the ester function of compound (46) with LiAlH₄, for example, prepares the compound (47), wherein s and t are independently as described, except that both s and t may not concurrently be 0. Compound (47) may be substituted for compound (2) and carried forward and reacted according to the alternate reaction pathways shown for compounds (2) or (7) in Scheme 1 to give the desired compound of formula (I).

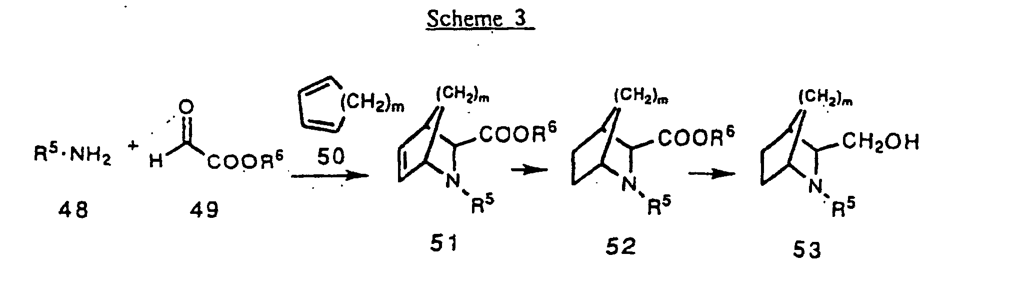

In accordance with Scheme 3 are prepared the compounds of Formula (II). The amine compound (48), wherein R⁵ is C₁-C₃-alkyl, a chiral auxiliary group (for example, (R)-1-phenylethyl, that is easily removed and replaced with H or the desired C₁-C₃-alkyl group), or an N-protecting group (such as BOC or CBZ, which may be subsequently removed and replaced with H or the desired C₁-C₃-alkyl group), is reacted with an appropriate glyoxylic ester (49), wherein R⁶ may be C₁-C₅-alkyl, in the presence of an appropriate C₅-C₇-cycloalka-1,3-diene compound (50), to give the variously substituted unsaturated compound (51), wherein m is 1, 2 or 3, and R⁵ and R⁶ are as described immediately above. Compound (51) is then reduced with H₂ in the presence of a noble metal catalyst, such as Pt or Pd/C to give compound (52), wherein m is as described above. Compound (52) is then reacted with a suitable ester reducing agent, such as LiAlH₄, LiBH₄, borane/THF or borane/methyl sulfide, for example, to afford the appropriately substituted alcohol compound (53), wherein m is as described above. Compound (53) may be substituted for compound (2) and carried forward and reacted according to the alternate reaction pathways shown for compounds (2) or (7) in Scheme 1 to give the desired compound of formula (III).

### In vitro Determination of Neuronal Nicotinic Receptor Binding Potencies, Selectivity and Functionality

For the purpose of identifying compounds as cholinergic agents which are capable of interacting with cholinergic channel receptors in the brain, a ligand-receptor binding assay was carried out as the initial screen. Compounds of the present invention were effective at interacting with neuronal nicotinic cholinergic receptors as.assayed *in vitro* for their ability to displace radioligand from neuronal nicotinic cholinergic channel receptors labeled with [³H]-cytisine ([³H]-CYT) (Protocol A below).

### A Protocol For Determination of Nicotinic Cholinergic Channel Receptor Binding Potencies of Ligands

Binding of [³H]-cytisine ([³H]-CYT) to nicotinic receptors was accomplished using crude synaptic membrane preparations from whole rat brain (Pabreza *et al., Molecular Pharmacol.,* **1990**, 39:9). Washed membranes were stored at-80°C prior to use. Frozen aliquots were slowly thawed and resuspended in 20 volumes of buffer (containing: 120 *mM* NaCl, 5 *mM* KCI, 2 *mM* MgCl₂, 2 *mM* CaCl₂ and 50 *mM* Tris-Cl, pH 7.4 @4°C). After centrifuging at 20,000x g for 15 minutes, the pellets were resuspended in 30 volumes of buffer. Homogenate (containing 125-150 µg protein) was added to triplicate tubes containing concentrations of test compound and [³H]-CYT (1.25 *nM*) in a final volume of 500 µL. Samples were incubated for 60 minutes at 4°C, then rapidly filtered through Whatman GF/B filters presoaked in 0.5% polyethyleneimine using 3 x 4 mL of ice-cold buffer. The filters are counted in 4 mL of Ecolume®(ICN). Nonspecific binding was determined in the presence of 10 µM (-)-nicotine and values were expressed as a percentage of total binding. IC₅₀ values were determined with the RS-1 (BBN) nonlinear least squares curve-fitting program and IC₅₀ values were converted to Ki values using the Cheng and Prusoff correction (Ki=IC₅₀/(1+ligand]/Kd of ligand). Alternately, data were expressed as a percentage of the total specific binding. The binding data (shown in Table 1) suggest that the compounds of the present invention have high affinity for the neuronal nicotinic cholinergic channel receptor.

**Table 1**

| Binding to Neuronal Nicotinic Receptors | |
|---|---|
| Ex. No | Binding (nM) |
| 3 | 6.8 |
| 7 | 11 |

### Examples

The present invention will be better understood by reference to the following examples, which are intended as an illustration of, and not a limitation upon, the scope of the invention as it is defined by the appended claims.

### Preparations of Starting Materials

Several starting materials are used repeatedly throughout the examples that follow. 1-Methyl-2-(S)-pyrrolidinemethanol was obtained from Aldrich Chemical Co. 1-Methyl-2-(R)-pyrrolidinemethanol was obtained from Fluka.

In the PCT Patent Application WO94 08992 of Abreo *et al.,* published April 28, 1994, and in Abreo, *et al. J. Med Chem.,* 3:, 817-825 (**1996**), are disclosed methods for preparing, *inter alia,* the (R) and (S) 1-BOC-2-(S)-pyrrolidinemethanol compounds, the (R) and (S) 1-BOC-2-(S)-azetidinemethanol compounds, and 1-Cbz-2-(R)-azetidinemethanol. 1-Cbz-2-(S)-azetidinemethanol is prepared from 2-(S)-azetidinecarboxylic acid using analogous procedures.

### Example 1 (Reference Example)

### Step 1 5-Bromo-6-chloro-3-(1-BOC-2-(S)-pyrrolidinylmethoxy)pyridine

To a solution of diethyl azodicarboxylate (1.89 mL, 12.0 mmol) in THF (30 mL) was added triphenylphosphine (3.15 g, 12.0 mmol) at 0°C, and the reaction mixture was stirred for 0.5 hour. 1-BOC-(S)-pyrrolidinemethanol (2.41 g, 12.0 mmol) and 5-bromo-6-chloropyridine-3-ol (2.09 g, 10.0 mmol) were then added. The reaction mixture was allowed to warm to room temperature overnight. The solvent was removed, and the residue was chromatographed on a silica gel column, eluting with EtOAc/hexane (1:5 and 1:2) to afford an oil (3.80 g, 97%). MS (CI/NH₃) m/z 391,393 (M+H)⁺. ¹H NMR (DMSO-d₆, 300 MHz) δ 1.65-2.05 (m, 4H), 3.20-3.35 (m, 2H), 3.95-4.15 (m, 3H), 7.98 (d, J = 2.9 Hz, 1H), 8.21 (d, J = 2.9 Hz, 1H).

### Example 2 (Reference Example)

### 5,6-Dichloro-3-(2-(R)-pyrrolidinylmethoxy)pyridine hydrochloride

### Step 2a. 5,6-Dichloro-3-(1-BOC-2-(R)-pyrrolidinylmethoxy)pyridine

To a solution of triphenylphosphine(2.6 g, 9.88 mmol) in THF (30 mL) was added diethyl azodicarboxylate (1.56 mL, 9.88 mmol) at 0°C, and the reaction mixture was stirred for 0.5 hour. 1-BOC-(R)-pyrrolidinemethanol (2.0 g, 9.88 mmol) and 5,6-dichloropyridine-3-ol (1.35 g, 8.23 mmol: prepared from 2-hydroxy-5-nitropyridine according to the procedure of V. Koch and S. Schnatterer, *Synthesis* **1990**, 499-501) were then added. The reaction mixture was slowly warmed up to room temperature overnight. The solvent was removed. and the residue was chromatographed on a silica gel column, eluting with EtOAc/hexane 1:6 to afford an oil (2.16 g). MS (CI/NH₃) m/z 347, 349 (M+H)⁺, 364, 366 (M+NH₄)⁺. ¹H NMR (CDCl₃, 300 MHz) δ 1.47 (s, 9H), 1.85-2.05 (m, 4H), 3.35-3.45 (m, 2H), 3.85-4.2 (m, 3H), 7.35-7.45 (br d, 1H), 8.02 (d, J = 2.5 Hz, 1H).

### Step 2b. 5,6-Dichloro-3-(2-(R)-pyrrolidinylmethoxy)pyridine hydrochloride

To 5,6-dichloro-3-(1-BOC-2-(R)-pyrrolidinylmethoxy)pyridine from step 2a (9.88 mmol) was added trifluoroacetic acid in methylene chloride (1:1, 20 mL) at 0°C, and the mixture was stirred for 30 minutes. The residue was neutralized with saturated KHCO₃ solution. then extracted with methylene chloride, which was dried over MgSO₄ and concentrated to afford the free base of the title compound (1.24 g, oil). The base (800 mg, 3.2 mmol) was converted to the salt by treatment with saturated hydrogen chloride in ether to give the title compound (398 mg). mp 250-252°C. MS (CI/NH₃) m/z 247, 249, 251 (M+H)⁺, 264, 266 (M+NH₄)⁺. ¹H NMR (D₂O, 300 MHz) δ 1.90-2.35 (m, 4H), 3.40-3.45 (m, 2H), 4.13 (m, 1H), 4.27 (m, 1H), 4.47 (m, 1H), 7.74 (d, J = 2.7 Hz, 1H), 8.07 (d, J = 2.7 Hz, 1H). Anal. Calcd. for C₁₀H₁₂N₂OCl₂• 1.0 HCl: C, 42.35; H, 4.62; N, 9.88. Found: C, 42.41; H, 4.49; N, 9.79. [α]²⁵_{D}=-11.1 °(c 1.0. MeOH).

### Example 3

### (1S,4R)-3-(S-)-(5-Bromo-6-chloro-3-pyridyloxymethyl)-N-methyl-2-azabicyclo[2.2.1]heptane hydrochloride

The tide compound was prepared as in example 6 below substituting 3-bromo-2-chloro-5-hydroxypyridine from step 1 for the 3-hydroxypyridine thereof: mp 237-238 °C; ¹H NMR (D₂O) δ 1.72 (m, 1H), 1.89 (m, 3H), 2.20(m, 2H), 2.63 (m, 1H), 3.02 (s, 3H), 3.44 (dd J=9,3 Hz, 1H), 4.05 (m, 1H), 4.19 (t, J=10 Hz, 1H), 4.44 (dd, J= 11,4 Hz, 1H), 7.91(d,J=3 Hz, 1H), 8.18 (d,J=3 Hz, 1H).

### Example 4 (Reference Example)

### (1R,4S)-3-(R)-(Hydroxymethyl)-N-methyl-2-azabicyclo[2.2.1]heptane

### 4a. (1R,4S)-3-(R)-Carboethoxy-2-azabicyclo[2.2.1] heptane hydrochloride

A mixture of (1R.4S)-3-(R)-carboethoxy-N-(S)-(-)-α-methylbenzyl-2-azabicyclo[2.2.1]hept-5-ene (2.40 g, 8.8 mmol, prepared according to the method of Stella *et al., Tetrahedron Lett.,* 31: 2603, **1990**) in ethanol (100 mL) and 20% Pd/C (dry, 1.2 g) was reduced under 4 atm of H₂ at room temperature for 12 hours. The solution was filtered and concentrated under vacuum to give the free base as an oil (1.33 g). MS (DCI/NH₃) m/e: 170 (M+H)⁺. ¹H NMR (CDCl₃, 300 MHz) δ:4.18 (q, 2H). 3.57 (br. s, 1H), 3.34 (s, 1H), 2.63 (br. s, 1H), 2.12 (m, 2H), 1.68-1.28 (m, 5H), 1.28 (t, 3H). The resultant oil was dissolved in methylene chloride (ca. 20 mL) and upon addition of HCl/diethyl ether (ca. 6.25 M) a white solid precipitated and was collected. The solid was then recrystallized from EtOH/Et₂O and dried under vacuum at 50°C to give the title compound (0.94 g, 52% yield). mp>200°C.

### 4b. (1R,4S)-3-(R)-Carboethoxy-N-t-butylcarboxy-2-azabicyclo[2.2.1]heptane

To the compound of step 4a (1.50 g, 7.3 mmol) in methylene chloride (20 mL) at room temperature under nitrogen was added triethylamine (1.0 mL, 7.3 mmol). After 5 min di-t-butyldicarbonate (1.84 mL. 8.0. mmol) was added. and the reaction was stirred for 18 hours. The reaction was quenched by the addition of aqueous pH 4 buffer, and the mixture was extracted with diethyl ether. The combined organic layers were dried over anhydrous sodium sulfate and concentrated under vacuum. The crude product was purified by flash chromatography (silica gel; ethyl acetate/hexane, 1:4; R_{f} = 0.45) to yield the title compound (1.49 g, 76%) as an oil. MS (DCI/NH₃) m/e: 270 (M+H)⁺. 287 (M+NH₄)⁺. ¹H NMR (CDCl₃, 300 MHz) δ: 4.28 (br. d, 1H), 4.18 (m, 2H), 3.78 (d, 1H), 2.67 (br. s, 1H), 1.94 (br. d, 1H), 1.80-1.40 (m, 5H), 1.44 (d, 9H), 1.28 (t, 3H).

### 4c. (1R,4S)-3-(R)-(Hydroxymethyl)-N-methyl-2-azabicyclo[2.2.1]heptane

To the compound of step 4b (1.40 g, 5.2 mmol) in anhydrous tetrahydrofuran (30 mL) under nitrogen was added lithium aluminum hydride (0.60 g, 1.56 mmol). The resultant solution was heated at reflux for 2 hours. cooled to room temperature and quenched by the careful addition of sodium sulfate decahydrate. Diethyl ether (50 mL) and celite were added, and the mixture was stirred at room temperature for 1 hour and filtered. The filtrate was concentrated under vacuum to give the title compound (0.79 g, 100%) as a white solid. mp 76-77°C. MS (DCI/NH₃) m/e: 142 (M+H)⁺. ¹H NMR (CDCl₃, 300 MHz) δ: 3.42 (dd, 1H), 3.41 (dd, 1H), 3.21 (br s, 1H), 2.36 (s, 3H), 2.15 (d, 1H), 1,98 (t, 1H), 1.94-1.85 (m, 1H), 1.80-1.70 (m, 1H), 1.60-1.52 (m, 1H), 1.38-1,20 (m, 3H).

### Example 5 (Reference Example)

### (1S,4R)-3-(S)-Hydroxymethyl)-N-methyl-2-azabicyclo[2.2.1]heptane

### 5a. (1S,4R)-3-(S)-Carboethoxy-2-azabicyclo[2.2.1]hept-2-ene hydrochloride

(1S,4R)-3-(S)-(Carboethoxy)-N-(R)-(+)-α-methylbenzyl-2-azabicyclo[2.2.1]hept-5-ene (11.0 g, 40.5 mmol, prepared by the method of Stella *et al., Terrahedron Lett.,* 31: 2603, 1990) was reacted according to the method described in Example 559a, to give the title compound (5.7 g, 68%) as a crystalline solid. mp >200 °C.

### 5b. (1S,4R)-3-(S)-(Carboethoxy)-N-t-butylcarboxy-2-azabicyclo[2.2.1]heptane

The compound from step 5a (5.0 g, 24.4 mmol), di-*t*-butyldicarbonate(5.8 g, 26.8 mmol) and triethylamine (3.4 g, 24.4 mmol) were reacted according to the method described in Example 559b to give the title compound (5.4 g, 82%) as an oil. MS (DCI/NH₃) m/e: 270(M+H)⁺, 287(M+NH₄)⁺. ¹H NMR (CDCl₃, 300 MHz)δ: 4.28 (d, 1H), 4.18 (m, 2H), 3.75 (d, 1H), 2.67 (br. s, 1H), 1.94 (m, 1H), 1.80-1.40 (m, 5H), 1.44 (d,9H),1.28 (t, 3H).

### 5c. (1S,4R)-3-(S)-(Hydroxymethyl)-N-methyl-2-azabicyclo[2.2.1]heptane

The compound from step 5b , (3.0 g, 11.1 mmol) and lithiumaluminum hydride (1.27 g, 33.4 mmol) were reacted according to the method described in Example 4c, gave the title compound (1.43 g, 92%) as a white solid. mp 75-76°C. MS(DCI/NH₃)m/e: 142(M+H)⁺, 140 (M-H)⁺. ¹H NMR (CDCl₃, 300 MHz) δ: 3.5-3.3 (m, 2H), 3.19 (br. s, 1H), 2.70 (br. s, 1H), 2.35 (s, 3H), 2.14 (d, 1H), 1.95 (t, 1H), 1.94-1.85 (m, 1H), 1.77-1.70 (m, 1H), 1.62-1.52 (m, 1H), 1.37-1.18 (m, 3H).

### Example 6 (Reference Example)

### (1S,4R)-3-(S)-(3-Pyridyloxymethyl)-N-methyl-2-azabicyclo[2.2.1]heptane dihydrochloride

To a solution of triphenylphosphine (1.78 g. 6.8 mmol) in tetrahydrofuran (40 mL) under nitrogen was added *t*-butylazodicarboxylate (1.56 g. 6.8 mmol). The solution was stirred at 0 °C for 20 minutes, and a THF solution (10 mL) of containing 3-hydroxypyridine (0.64 g, 6.8 mmol) and the compound (1S,4R)-3-(S)-(hydroxymethyl)-N-methyl-2-azabicyclo[2.2.1]heptane (0.64 g, 4.5 mmol, from Example 5c) was added. The reaction was allowed to warm to room temperature, stirred for 72 hours, then concentrated under vacuum. The residue was taken up in an aqueous solution of hydrochloric acid (10%, 100 mL) containing a small amount (*ca* .20 mL) of methylene chloride and stirred for 2 hours. The solution was washed with methylene chloride. The aqueous layer was adjusted to pH 12 with 10% aqueous sodium hydroxide and exacted with methylene chloride. The combined organic extracts were dried over anhydrous sodium sulfate and concentrated under vacuum. The crude product was purified by flash chromatography (silica gel; methylene chloride/ethyl acetate/methanol/ammonium hydroxide. 50:50:4:1; R_{f} = 0.3 1) to give an oil (80 mg, 8.0%). The oil was dissolved in methylene chloride, and addition of HCl/diethyl ether (6.25M, 15mL) gave a white solid which was collected and dried under vacuum at 50 °C to give the title compound as a deliquescent white solid. MS (DCI/NH₃) m/e: 219 (M+H)⁺. ¹H NMR (DMSO-*d*₆, 300MHz) δ: 11.35 (br. s, 1H), 8.75 (d, 1H), 8.53 (d, 1H), 8.18 (m; 1H), 7.38 (dd, 1H), 4.48 (m, 2H), 3.88 (br. s, 1H), 3.43-73.36 (m, 1H), 2.85 (d, 3H), 2.45 (br. s, 1H), 2.15 (d, 1H), 1.95 (m, 1H), 1.80-1.60 (m, 4H). IR (KBr) 3260, 3180, 1650, 1550, 1380, 1000, 810 cm-1; Anal. calc. for C₁₃H₂₀Cl₂N₂O·0.7 NH₄Cl·0.3H₂O: C, 46.74 H, 7.06; N, 11.32. Found: C,46.90; H, 7.33; N, 11.24.

### Example 7

### (1S,4R)-3-(S)-(5,6-Dichloro-3-pyridyloxymethyl)-N-methyl-2-azabicylo[2.2.1]heptane hydrochloride

The title compound was prepared as in example 6 substituting 2,3-dichloro-5-hydroxypyridine from step 2 for the 3-hydroxypyridine thereof: mp 235-237°C; ¹H NMR (D₂O) δ 1.67 (m, 1H), 1.86 (m, 3H), 2.08 (m, 2H), 2.66 (br s, 1H), 2.98 (s, 3H), 3.45 (m, 1H), 4.05 (br s, 1H), 4.21 (t, J=10 Hz, 1H), 4.45 (dd, J=10,4 Hz, 1H), 7.76 (d,J=3 Hz, 1H), 8.10 (d. J=3 Hz, 1H); MS (Cl/NH₃) m/z 287 (M+H)+; Anal. Calcd for C₁₃H₁₆N₂OCl₂•HCl: C, 48.24; H, 5.29; N, 8.65. Found: C, 48.10; H, 5.25; N, 8.43.

## Claims

1. Compound having the formula (I) : 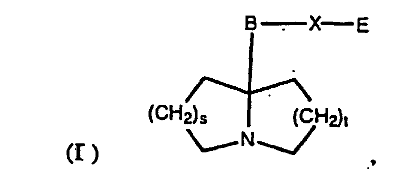 or a pharmaceutically acceptable salt or prodrug thereof, wherein
B is 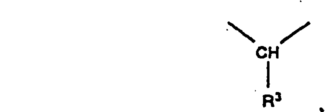 wherein R³ is H or C₁-C₆=alkyl;
X is selected from the group consisting of oxygen and sulfur; and
E is 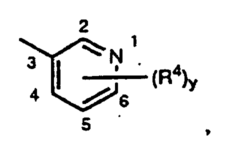 wherein
y is an integer selected from 2 and 3, with the requirements that
(a) when y=2,
R⁴ is selected from the group having substituents at the 2,4-, 2,5-, 2,6-, 4,5-, 4,6- and 5,6- positions of the pyridine ring wherein
a 2-position substituent is selected from the group consisting of
(i) -Br,
(ii) -Cl,
(iii) -F,
(iv) -OH,
(v) -NH₂,
(vi) -C₁-C₄-alkyl, and
(vii) -C₁-C₃-alkoxy; and
a substituent at the 4-, 5- and 6-posidon of the pyridine ring is selected from the group consisting of
(i) -Br,
(ii) -Cl,
(iii) -F,
(iv) -OH,
(v) -C₁-C₄-alkyl,
(vi) -CN,
(vii) -CH₂F,
(viii) -CHF₂,
(ix) -CF₃,
(x) -NO₂,
(xi) -CH₂OH,
(xii) -CH₂CN,
(xiii) -C₁-C₃-alkoxy,
(xiv) -NH₂,
(xv) -NH-CHO,
(xvi) -NH-C(O)-(C₁-C₃-alkyl),
(xvii) -N(C₁-C₃-alkyl)-C(O)-(C₁-C₃-alkyl),
(xviii) -NH-(C₁-C₃-alkyl),
(xix) -NH-CH₂-phenyl,
(xx) -N(C₁-C₃-alkyl)₂,
(xxi) -C(O)-OH,
(xxii) -C(O)-O-C₁-C₃-alkyl,
(xxiii) -C(O)-NH₂,
(xxiv) -C(O)-NH-C₁-C₃₋alkyl,
(xxv) -C(O)-NH-CH₂-phenyl, and
(xxvi) -O-C(O)-(C₁-C₃-alkyl); and
with the requirement that when there is no 2-position substitutent, then one R⁴ substituent must be selected from the group consisting of:
(i) -Br,
(ii) -Cl,
(iii) -F,
(iv) -CN,
(v) -CH₂OH,
(vi) -C₁-C₄-alkyl, and
(b) when y=3,
R⁴ is selected from the group having substituents at the 2,4,5-, 2,4,6-, 2,5,6- and 4,5,6- positions of the pyridine ring wherein
a 2-position substituent is selected from the group consisting of
(i) -Br,
(ii) -Cl,
(ii) -F,
(iv) -OH,
(v) -C₁-C₄-alkyl, and
(vi) -C₁-C₃ alkoxy; and
a substituent at the 4-, 5- and 6-position of the pyridine ring is selected from the group consisting of
(i) -Br,
(ii) -Cl,
(iii) -F,
(iv) -OH,
(v) -C₁-C₄-alkyl,
(vi) -CN,
(vii) -CH₂F,
(viii) -CHF₂,
(ix) -CF₃,
(x) -NO₂,
(xi) -CH₂OH,
(xii) -CH₂CN,
(xiii) -C₁-C₃-alkoxy,
(xiv) -NH₂,
(xv) -NH-CHO,
(xvi) -NH-C(O)-(C₁-C₃-alkyl),
(xvii) -N(C₁-C₃-alkyl)-C(O)-(C₁-C₃-alkyl),
(xviii) -NH-(C₁-C₃-alkyl),
(xix) -NH-CH₂-phenyl,
(xx) -N(C₁-C₃-alkyl)₂,
(xxi) -C(O)-OH,
(xxii) -C(O)-O-C₁-C₃-alkyl,
(xxiii) -C(O)-NH₂,
(xxiv) -C(O)-NH-C₁-C₃-alkyl,
(xxv) -C(O)-NH-CH₂-phenyl, and
(xxvi) -O-C(O)-(C₁-C₃-alkyl); and
with the requirement that when there is no 2-position substitutent, then two R⁴ substituents must be selected from the group consisting of
(i) -Br,
(ii) -Cl,
(iii) -F,
(iv) -CN,
(v) -CH₂OH,
(vi) -C₁-C₄-alkyl,
and s and t are integers independently selected from the group consisting of 0, 1 and 2, with the requirement that both s and t may not concurrently be 0.

2. The compound according to Claim 1, or a pharmaceutically acceptable salt or prodrug thereof, wherein X is oxygen.

3. Compound having the formula (II): 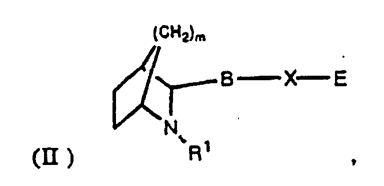 or a pharmaceutically acceptable salt or prodrug thereof, wherein m is selected from the group consisting of 1 and 2,
R¹ is selected from the group consisting of H, allyl and C₁-C₆ alkyl,
B is 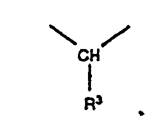 wherein R³ is H or C₁-C₆=alkyl;
X is selected from the group consisting of oxygen and sulfur; and
E is 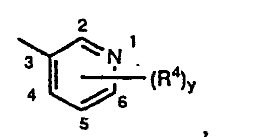 wherein
y is an integer selected from : 2 and 3, with the requirements that
(a) when y=2,
R⁴ is selected from the group having substituents at the 2,4-, 2,5-, 2,6-, 4,5-, 4,6- and 5,6- positions of the pyridine ring wherein
a 2-position substituent is selected from the group consisting of
(i) -Br,
(ii) -Cl,
(iii) -F,
(iv) -OH,
(v) -NH₂,
(vi) -C₁-C₄-alkyl, and
(vii) -C₁-C₃-alkoxy; and
a substituent at the 4-, 5- and 6-position of the pyridine ring is selected from the group consisting of
(i) -Br,
(ii) -Cl,
(iii) -F,
(iv) -OH,
(v) -C₁-C₄-alkyl,
(vi) -CN,
(vii) -CH₂F,
(viii) -CHF₂,
(ix) -CF₃,
(x) -NO₂,
(xi) -CH₂OH,
(xii) -CH₂CN,
(xiii) -C₁-C₃-alkoxy,
(xiv) -NH₂,
(xv) -NH-CHO,
(xvi) -NH-C(O)-(C₁-C₃-alkyl),
(xvii) -N(C₁-C₃-alkyl)-C(O)-(C₁-C₃-alkyl),
(xviii) -NH-(C₁-C₃-alkyl),
(xix) -NH-CH₂-phenyl,
(xx) -N(C₁-C₃-alkyl)₂,
(xxi) -C(O)-OH,
(xxii) -C(O)-O-C₁-C₃-alkyl,
(xxiii) -C(O)-NH₂,
(xxiv) -C(O)-NH-C₁-C₃-alkyl,
(xxv) -C(O)-NH-CH₂-phenyl, and
(xxvi) -O-C(O)-(C₁-C₃-alkyl); and
with the requirement that when there is no 2-position substitutent, then one R⁴ substituent must be selected from the group consisting of:
(i) -Br,
(ii) -Cl,
(iii) -F,
(iv) -CN,
(v) -CH₂OH,
(vi) -C₁-C₄-alkyl, and
(b) when y=3,
R⁴ is selected from the group having substituents at the 2,4,5-, 2,4,6-, 2,5,6- and 4,5,6- positions of the pyridine ring wherein
a 2-position substituent is selected from the group consisting of
(i) -Br,
(ii) -Cl,
(ii) -F,
(iv) -OH,
(v) -C₁-C₄-alkyl, and
(vi) -C₁-C₃ alkoxy; and
a substituent at the 4-, 5- and 6-position of the pyridine ring is selected from the group consisting of
(i) -Br,
(ii) -Cl,
(iii) -F,
(iv) -OH,
(v) -C₁-C₄-alkyl,
(vi) -CN,
(vii) -CH₂F,
(viii) -CHF₂,
(ix) -CF₃,
(x) -NO₂,
(xi) -CH₂OH,
(xii) -CH₂CN,
(xiii) -C₁-C₃-alkoxy,
(xiv) -NH₂,
(xv) -NH-CHO,
(xvi) -NH-C(O)-(C₁-C₃-alkyl),
(xvii) -N(C₁-C₃-alkyl)-C(O)-C₁-C₃-alkyl),
(xviii) -NH-(C₁-C₃-alkyl),
(xix) -NH-CH₂-phenyl,
(xx) -N(C₁-C₃-alkyl)₂,
(xxi) -C(O)-OH,
(xxii) -C(O)-O-C₁-C₃-alkyl,
(xxiii) -C(O)-NH₂,
(xxiv) -C(O)-NN-C₁-C₃-alkyl,
(xxv) -C(O)-NH-CH₂-phenyl, and
(xxvi) -O-C(O)-(C₁-C₃-alkyl); and
with the requirement that when there is no 2-position subsututent, then two R⁴ substituents must be selected from the group consisting of
(i) -Br,
(ii) -Cl,
(iii) -F,
(iv) -CN,
(v) -CH₂OH,
(vi) C₁-C₄-alkyl.

4. The compound according to Claim 3, or a pharmaceutically acceptable salt or prodrug thereof, which is selected from the group consisting of:
(1S,4R)-3-(S)-(5,6-dichloro-3-pyridyloxymethyl)-N-methyl-2-azabicyclo[2.2.1]heptane;
(1S,4R)-3-(S)-(5-bromo-6-chloro-3-pyridyloxymethyl)-N-methyl-2-azabicyclo[2.2.1]heptane.

5. The compound according to Claim 3, or a pharmaceutically acceptable salt or prodrug thereof, wherein X is oxygen.

6. A pharmaceutical composition for controlling synaptic transmission, comprising a pharmaceutically acceptable carrier and a therapeutically effective amount of the compound of any one of claims 1 to 6, or the pharmaceutically acceptable salts and prodrugs thereof.

7. Use of the compound any one of claims 1 to 6, or the pharmaceutically acceptable salts prodrugs thereof for manufacturing a medicament for selectively controlling synaptic transmission by administration to a human or veterinary patient in need of such treatment a therapeutically-effective amount of said compound or salt and prodrug thereof.

8. Use of a compound according to any one of claims 1 to 6 for manufacturing a medicament for treating dementias, attention-deficit disorder, anxiety associated with cognitive impairment or substance abuse withdrawal **characterized by** decreased cholinergic function by administration to a human or veterinary patient in need of such treatment a therapeutically-effective amount of said compound.

## Patentansprüche

1. Verbindung mit der Formel (I): 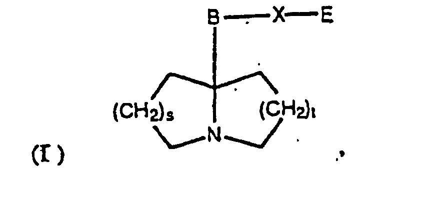 oder ein pharmazeutisch verträgliches Salz oder Prodrug davon, worin
B 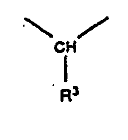 ist,
worin R³ H oder C₁-C₆-Alkyl ist;
X ist gewählt aus der Gruppe bestehend aus Sauerstoff und Schwefel; und
E ist 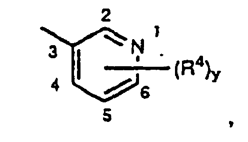 worin
y eine eine ganze Zahl ist gewählt aus 1, 2 und 3, mit den Bedingungen, daß
(a) wenn y=2,
R⁴ gewählt ist aus der Gruppe, die Substituenten an den 2,4-, 2,5-, 2,6-, 4,5-, 4,6- und 5,6-Positionen des Pyridinrings hat, worin
ein Substituent an der 2-Position gewählt ist aus der Gruppe bestehend aus
(i) -Br,
(ii) -Cl,
(iii) -F,
(iv) - -OH,
(v) -NH₂,
(vi) -C₁-C₄-Alkyl, und
(vii) -C₁-C₃-Alkoxy; und
ein Substituent an der 4-, 5- und 6-Position des Pyridinrings ist gewählt aus der Gruppe bestehend aus
(i) -Br,
(ii) -Cl,
(iii) -F,
(iv) -OH,
(v) -C₁-C₄-Alkyl,
(vi) -CN,
(vii) -CH₂F,
(viii) -CHF₂,
(ix) -CF₃,
(x) -NO₂,
(xi) -CH₂OH,
(xii) -CH₂CN,
(xiii) -C₁-C₃-Alkoxy,
(xiv) -NH₂,
(xv) -NH-CHO,
(xvi) -NH-C(O)-(C₁-C₃-Alkyl),
(xvii) -N (C₁-C₃-Alkyl ) -C(O)- (C₁-C₃-Alkyl),
(xviii) -NH-(C₁-C₃-Alkyl),
(xix) -NH-CH₂-Phenyl,
(xx) -N(C₁-C₃-Alkyl)₂,
(xxi) -C(O)-OH,
(xxii) -C (O) -O-C₁-C₃-Alkyl,
(xxiii) -C(O)-NH₂,
(xxiv) -C(O)-NH-C₁-C₃-Alkyl,
(xxv) -C(O)-NH-CH₂-Phenyl, und
(xxvi) -O-C(O)-(C₁-C₃-Alkyl); und
mit der Bedingung, daß, wenn es keinen Substituenten an der 2-Position gibt, dann muß ein R⁴ Substituent gewählt sein aus der Gruppe bestehend aus:
(i) -Br,
(ii) -Cl,
(iii) -F,
(iv) -CN,
(v) -CH₂OH,
(vi) -C₁-C₄-Alkyl, und
(b) wenn y=3,
R⁴ gewählt ist aus der Gruppe, die Substituenten an den 2,4,5-, 2,4,6-, 2,5,6- und 4,5,6-Positionen des Pyridinrings: hat, worin ein Substituent an der 2-Position gewählt ist aus der Gruppe bestehend aus
(i) -Br,
(ii) -Cl,
(ii) -F,
(iv) -OH,
(v) -C₁-C₄-Alkyl, und
(vi) -C₁-C₃ Alkoxy; und
ein Substituent an der 4-, 5- und 6-Position des Pyridinrings ist gewählt aus der Gruppe bestehend aus
(i) -Br,
(ii) -Cl,
(iii) -F,
(iv) -OH,
(v) -C₁-C₄-Alkyl,
(vi) -CN,
(vii) -CH₂F,
(viii) -CHF₂;
(ix) -CF₃,
(x) -NO₂,
(xi) -CH₂OH,
(xii) -CH₂CN,
(xiii) -C₁-C₃-Alkoxy,
(xiv) -NH₂,
(xv) -NH-CHO,
(xvi) -NH-C(O)-(C₁-C₃-Alkyl),
(xvii) -N(C₁-C₃-Alkyl)-C(O)-(C₁-C₃-Alkyl),
(xviii) -NH-(C₁-C₃-Alkyl),
(xix) -NH-CH₂-Phenyl,
(xx) -N(C₁-C₃-Alkyl)₂,
(xxi) -C(O)-OH,
(xxii) -C(O)-O-C₁-C₃-Alkyl,
(xxiii) -C(O)-NH₂,
(xxiv) -C(O)-NH-C₁-C₃-Alkyl,
(xxv) -C(O)-NH-CH₂-Phenyl, und
(xxvi) -O-C(O)-(C₁-C₃-Alkyl); und
mit der Bedingung, daß wenn es keinen Substituenten. an der 2-Position gibt, dann müssen zwei R⁴ Substituenten gewählt sein aus der Gruppe bestehend aus:
(i) -Br,
(ii) -Cl,
(iii) -F,
(iv) -CN,
(v) -CH₂OH,
(vi) -C₁-C₄-Alkyl,
und s und t sind ganze Zahlen, unabhängig gewählt aus der Gruppe bestehend aus 0, 1 und 2, mit der Bedingung, daß sowohl s als auch t nicht gleichzeitig 0 sein können.

2. Die Verbindung gemäß Anspruch 1 oder ein pharmazeutisch verträgliches Salz oder Prodrug davon, worin X Sauerstoff ist.

3. Verbindung mit der Formel (II): 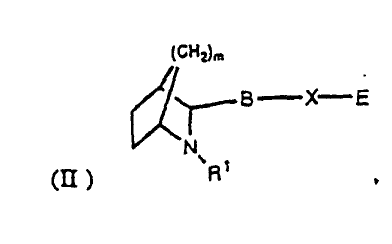 oder ein pharmazeutisch verträgliches Salz oder Prodrug davon, worin m gewählt ist aus der Gruppe bestehend aus 1 und 2,
R¹ ist gewählt aus der Gruppe bestehend aus H, Allyl und C₁-C₆-Alkyl,
B ist 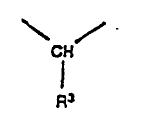 worin R³ H oder C₁-C₆-Alkyl ist;
X ist gewählt aus der Gruppe bestehend aus Sauerstoff und Schwefel; und
E ist 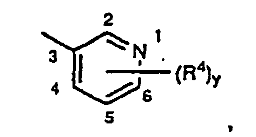 worin
y eine ganze Zahl ist, gewählt aus 1, 2 und 3, mit den Bedingungen, daß
(a) wenn y=2,
R⁴ gewählt ist aus der Gruppe, die Substituenten an den 2,4-, 2,5-, 2,6-, 4,5-, 4,6- und 5,6-Positionen des Pyridinrings hat, worin ein Substituent an der 2-Position gewählt ist aus der Gruppe bestehend aus
(i) -Br,
(ii) -Cl,
(iii) -F,
(iv) -OH,
(v) -NH₂,
(vi) -C₁-C₄-Alkyl, und
(vii) -C₁-C₃-Alkoxy; und
ein Substituent an der 4-, 5- und 6-Position des Pyridinrings ist gewählt aus der Gruppe bestehend aus
(i) -Br,
(ii) -Cl,
(iii) -F,
(iv) -OH,
(v) -C₁-C₄-Alkyl,
(vi) -CN,
(vii) -CH₂F,
(viii) -CHF₂,
(ix) -CF₃,
(x) -NO₂,
(xi) -CH₂OH,
(xii) -CH₂CN,
(xiii) -C₁-C₃-Alkoxy,
(xiv) -NH₂,
(xv) -NH-CHO,
(xvi) -NH-C(O)-(C₁-C₃-Alkyl),
(xvii) -N(C₁-C₃-Alkyl)-C(O)-(C₁-C₃-Alkyl),
(xviii) -NH-(C₁-C₃-Alkyl),
(xix) -NH-CH₂-Phenyl,
(xx) -N(C₁-C₃-Alkyl)₂,
(xxi) -C(O)-OH,
(xxii) -C(O)-O-C₁-C₃-Alkyl,
(xxiii) -C(O)-NH₂,
(xxiv) -C(O)-NH-C₁-C₃-Alkyl,
(xxv) -C(O)-NH-CH₂-Phenyl, und
(xxvi) -O-C(O)-(C₁-C₃-Alkyl); und
mit der Bedingung, daß, wenn es keinen Substituent an der 2-Position gibt, dann muß ein R⁴ Substituent gewählt sein aus der Gruppe bestehend aus:
(i) -Br,
(ii) -Cl,
(iii) -F,
(iv) -CN,
(v) -CH₂OH,
(vi) -C₁-C₄-Alkyl, und
(b) wenn y=3,
R⁴ gewählt ist aus der Gruppe, die Substituenten an den 2,4,5-, 2,4,6-, 2,5,6- und 4,5,6-Positionen des Pyridinrings hat, worin ein Substituent an der 2-Position gewählt ist aus der Gruppe bestehend aus
(i) -Br,
(ii) -Cl,
(ii) -F,
(iv) -OH,
(v) -C₁-C₄-Alkyl, und
(vi) -C₁-C₃ Aloxy; und
ein Substituent an der 4-, 5- und 6-Position des Pyridinrings ist gewählt aus der Gruppe bestehend aus
(i) -Br,
(ii) -Cl,
(iii) -F,
(iv) -OH,
(v) -C₁-C₄-Alkyl,
(vi) -CN,
(vii) -CH₂F,
(viii) -CHF₂,
(ix) -CF₃,
(x) -NO₂,
(xi) -CH₂OH,
(xii) -CH₂CN,
(xiii) -C₁-C₃-Alkoxy,
(xiv) -NH₂,
(xv) -NH-CHO,
(xvi) -NH-C(O)-(C₁-C₃-Alkyl),
(xvii) -N(C₁-C₃-Alkyl)-C(O)-(C₁-C₃-Alkyl),
(xviii) -NH-(C₁-C₃-Alkyl),
(xix) -NH-CH₂-Phenyl,
(xx) -N(C₁-C₃-Alkyl)₂,
(xxi) -C(O)-OH,
(xxii) -C(O)-O-C₁-C₃-Alkyl,
(xxiii) -C(O)-NH₂,
(xxiv) -C(O)-NH-C₁-C₃-Alkyl,
(xxv) -C(O)-NH-CH₂-Phenyl, und
(xxvi) -O-C(O)-(C₁-C₃-Alkyl); und
mit der Bedingung, daß, wenn es keinen Substituenten an der 2-Position gibt, dann müssen zwei R⁴ Substituenten gewählt sein aus der Gruppe bestehend aus
(i) -Br,
(ii) -Cl,
(iii) -F,
(iv) -CN,
(v) -CH₂OH,
(vi) -C₁-C₄-Alkyl.

4. Die Verbindung gemäß Anspruch 3 oder ein pharmazeutisch verträgliches Salz oder Prodrug davon, die gewählt ist aus der Gruppe bestehend aus:
(1S,4R)-3-(S)-(5,6-Dichlor-3-pyridyloxymethyl)-N-methyl-2-azabicyclo[2.2.1]heptan;
(1S,4R)-3-(S)-(5-Brom-6-chlor-3-pyridyloxymethyl)-N-methyl-2-azabicyclo[2.2.1]heptan.

5. Die Verbindung gemäß Anspruch 3 oder ein pharmazeutisch verträgliches Salz oder Prodrug davon, worin X Sauerstoff ist.

6. Eine pharmazeutische Zusammensetzung zur Kontrolle der synaptischen Übertragung, die einen pharmazeutisch verträglichen Träger und eine therapeutisch wirksame Menge einer Verbindung von irgendeinem der Ansprüche 1 bis 6 umfaßt, oder die pharmazeutisch verträglichen Salze und Prodrugs davon.

7. Verwendung der Verbindung von irgendeinem der Ansprüche 1 bis 6, oder der pharmazeutisch verträglichen Salze, Prodrugs davon, zur Herstellung eines Medikaments zur selektiven Kontrolle der synaptischen Übertragung durch Verabreichen einer therapeutisch wirksamen Menge der Verbindung oder eines Salzes oder Prodrugs davon, an einen Menschen oder einen Veterinärpatienten, der eine solche Behandlung benötigt.

8. Verwendung einer Verbindung gemäß irgendeinem der Ansprüche 1 bis 6 zur Herstellung eines Medikaments zur Behandlung von Dementia, Aufmerksamkeitsdefizit-Störung, Angst in Zusammenhang mit kognitiver Beeinträchtigung oder Entzug bei Substanzmißbrauch, **gekennzeichnet durch** eine verminderte cholinerge Funktion, **durch** Verabreichen einer therapeutisch wirksamen Menge der Verbindung an einen Menschen oder Veterinärpatienten, der eine solche Behandlung benötigt.

## Revendications

1. Composé ayant la formule (I) 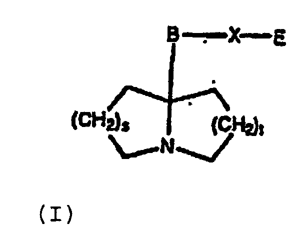 ou son sel ou promédicament pharmaceutiquement acceptable dans lequel
B est 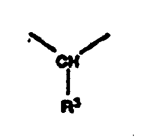 Dans lequel R³ est un H ou un alkyle en C₁-C₆;
X est choisi dans le groupe composé d'oxygène et de soufre ; et
E est 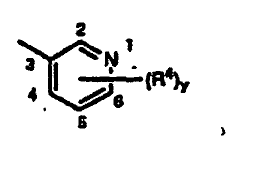 Dans lequel
y est un nombre entier choisi parmi 1, 2 et 3 à condition que
(a) quand y = 2,
R⁴ soit choisi dans le groupe ayant des substituants en position 2,4 - 2,5-, 2,6-, 4,5-, 4,6- et 5,6- de l'anneau de pyridine dans lequel
Un substituant en position 2 est choisi dans le groupe composé de :
(i) -Br
(ii) -Cl
(iii) -F
(iv) -OH
(v) -NH₂
(vi) -alkyle en C₁-C₄, et
(vii) -alcoxy en C₁-C₃ et
un substituant en position -4, -5, et 6 de l'anneau de pyridine est choisi dans le groupe composé de
(i) -Br
(ii) -CI
(iii) -F
(iv) -OH
(v) -alkyle en C₁-C₄
(vi) -CN
(vii) -CH₂F
(viii) -CHF₂
(ix) -CF₃
(x) -NO₂
(xi) -CH₂OH
(xii) -CH₂CN
(xiii) -alcoxy en C₁-C₃
(xiv) -NH₂
(xv) -NH-CHO
(xvi) -NH-C(O)-(alkyle en C₁-C₃)
(xvii) -N(alkyle en C₁-C₃)-C(O)-(alkyle en C₁-C₃)
(xviii) -NH-(alkyle en C₁-C₃)
(xix) -NH-CH₂-phényl
(xx) -N(alkyle en C₁-C₃)₂
(xxi) -C(O)-OH
(xxii) -C(O)-O-alkyle en C₁-C₃
(xxiii) -C(O)-NH₂
(xxiv) -C(O)-NH-alkyle en C₁-C₃
(xxv) -C(O)-NH-CH₂-phényle et
(xxvi) -O-C(O)-(alkyle en C₁-C₃) et
à condition que lorsqu'il n'y a pas de substituant en position 2, alors un substituant R⁴ doit être choisi dans le groupe composé de
(i) -Br
(ii) -Cl
(ii) -Cl
(iii) -F
(iv) -CN
(v) -CH₂OH
(vi) -alkyle en C₁-C₄ et
(b) quand y = 3
R⁴ soit choisi dans le groupe ayant des substituants en positions 2,4,5 - 2,4,6 - 2,5,6 et 4,5,6 de l'anneau de pyridine dans lequel
Un substituant en position 2 est choisi dans le groupe composé de
(i) -Br
(ii) -Cl
(iii) -F
(iv) -OH
(v) -alkyle en C₁-C₄ et
(vi) -alcoxy en C₁-C₃ et
un substituant en position 4, 5 et 6 de l'anneau de pyridine est choisi dans le groupe composé de
(i) -Br
(ii) -Cl
(iii) -F
(iv) -OH
(v) -alkyle en C₁-C₄
(vi) -CN
(vii) -CH₂F
(viii) -CHF₂
(ix) -CF₃
(x) -NO₂
(xi) -CH₂OH
(xii) -CH₂CN
(xiii) -alcoxy en C₁-C₃
(xiv) -NH₂
(xv) -NH-CHO
(xvi) -NH-C(O)-(alkyle en C₁-C₃)
(xvii) -N(alkyle en C₁-C₃)-C(O)-(alkyle en C₁-C₃)
(xviii) -NH-(alkyle en C₁-C₃)
(xix) -NH-CH₂-phényl
(xx) -N(alkyle en C₁-C₃)₂
(xxi) -C(O)-OH
(xxii) -C(O)-O-alkyle en C₁-C₃
(xxiii) -C(O)-NH₂
(xxiv) -C(O)-NH-alkyle en C₁-C₃
(xxv) -C(O)-NH-CH₂-phényle et
(xxvi) -O-C(O)-(alkyle en C₁-C₃) et
à condition que lorsqu'il n'y a pas de substituant en position 2, alors un substituant R⁴ doit être choisi dans le groupe composé de
(i) - Br
(ii) -Cl
(iii) -F
(iv) -CN
(v) -CH₂OH
(vi) -alkyle en C₁-C₄
et s et t sont des nombres entiers choisis indépendamment parmi 0, 1 et 2 à condition que s et t ne puissent pas être simultanément 0.

2. Composé selon la revendication 1, ou son sel ou promédicament pharmaceutiquement acceptable dans lequel X est de l'oxygène.

3. Composé ayant la formule (II) 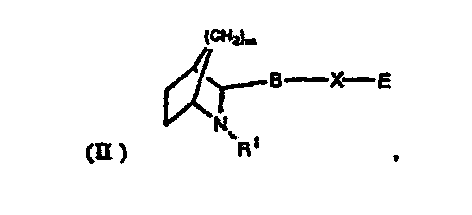 Ou son sel ou promédicament pharmaceutiquement acceptable, dans lequel m est choisi dans le groupe composé de 1 et 2 ;
R¹ est choisi dans le groupe composé de H, d'allyle et d'alkyle en C₁-C₆;
B est 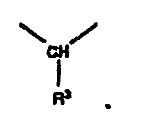 Dans laquelle R³ est un H ou un alkyle en C₁-C₆ ;
x est choisi dans le groupe composé d'oxygène et de soufre ; et
E est 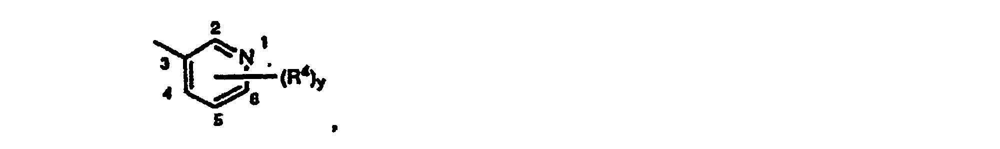 Dans laquelle y est un nombre entier choisi parmi 1, 2 et 3 à condition que
a. quand y = 2
R⁴ soit choisi dans le groupe ayant des substituants en positions 2,4 - 2,5 - 2,6 - 4,5 - 4,6 - et 5,6 de l'anneau de pyridine dans lequel
Un substituant en position 2 est choisi dans le groupe composé de
(i) -Br
(ii) -Cl
(iii) -F
(iv) -OH
(v) -NH₂
(vi) -alkyle en C₁-C₄, et
(vii) -alcoxy en C₁-C₃ et
un substituant en position -4, -5, et 6 de l'anneau de pyridine est choisi dans le groupe composé de
(i) -Br
(ii) -Cl
(iii) -F
(iv) -OH
(v) -alkyle en C₁-C₄
(vi) -CN
(vii) -CH₂F
(viii) -CHF₂
(ix) -CF₃
(x) -NO₂
(xi) -CH₂OH
(xii) -CH₂CN
(xiii) -alcoxy en C₁-C₃
(xiv) -NH₂
(xv) -NH-CHO
(xvi) -NH-C(O)-(alkyle en C₁-C₃)
(xvii) -N(alkyle en C₁-C₃)-C(O)-(alkyle en C₁-C₃)
(xviii) -NH-(alkyle en C₁-C₃)
(xix) -NH-CH₂-phényle
(xx) -N(alkyle en C₁-C₃)₂
(xxi) -C(O)-OH
(xxii) -C(O)-O-alkyle en C₁-C₃
(xxiii) -C(O)-NH₂
(xxiv) -C(O)-NH-alkyle en C₁-C₃
(xxv) -C(O)-NH-CH₂-phényle et
(xxvi) -O-C(O)-(alkyle en C₁-C₃) et
à condition que lorsqu'il n'y a pas de substituant en position 2, alors un substituant R⁴ doit être choisi dans le groupe composé de
(i) - Br
(ii) -Cl
(iii) -F
(iv) -CN
(v) -CH₂OH
(vi) -alkyle en C₁-C₄ et
(b) quand y = 3
R⁴ est choisi dans le groupe ayant des substituants en positions 2,4,5 - 2,4,6 - 2,5,6 et 4,5,6 de l'anneau de pyridine dans lequel
Un substituant en position 2 est choisi dans le groupe composé de
(i) -Br
(ii) -Cl
(iii) -F
(iv) -OH
(v) -alkyle en C₁-C₄ et
(vi) -alcoxy en C₁-C₃ et
un substituant en position 4, 5 et 6 de l'anneau de pyridine est choisi dans le groupe composé de
(i) -Br
(ii) -Cl
(iii) -F
(iv) -OH
(v) -alkyle en C₁-C₄
(vi) -CN
(vii) -CH₂F
(viii) -CHF₂
(ix) -CF₃
(x) -NO₂
(xi) -CH₂OH
(xii) -CH₂CN
(xiii) -alcoxy en C₁-C₃
(xiv) -NH₂
(xv) -NH-CHO
(xvi) -NH-C(O)- (alkyle en C₂-C₃)
(xvii) -N(alkyle en C₁-C₃) -C(O)- (alkyle en C₁-C₃)
(xviii) -NH-(alkyle en C₁-C₃)
(xix) -NH-CH₂-phényle
(xx) -N(alkyle en C₁-C₃)₂
(xxi) -C(O)-OH
(xxii) -C(O)-O-alkyle en C₁-C₃
(xxiii) -C(O)-NH₂
(xxiv) -C(O)-NH-alkyle en C₁-C₃
(xxv) -C (O) -NH-CH₂-phényle et
(xxvi) -O-C(O)-(alkyle en C₁-C₃) et
à condition que lorsqu'il n'y a pas de substituant en position 2, alors un substituant R⁴ doit être choisi dans le groupe composé de
(i) - Br
(ii) -Cl
(iii) -F
(iv) -CN
(v) -CH₂OH
(vi) -alkyle en C₁-C₄

4. Composé selon la revendication 3, ou son sel ou promédicament pharmaceutiquement acceptable, choisi dans le groupe composé de
(1S,4R)-3(S)-(5,6-dichloro-3-pyridyloxyméthyl)-N-méthyl-2-azabicyclo[2,2,1]heptane,
(1S,4R)-3-(S)-(5-bromo-6-chloro-3-pyridyloxyméthyl)-N-méthyl-2-azabicyclo[2,2,1]heptane

5. Composé selon la revendication 3, ou son sel ou promédicament pharmaceutiquement acceptable dans lequel X est de l'oxygène

6. Composition pharmaceutique permettant de contrôler la transmission synaptique comprenant un support pharmaceutiquement acceptable et une quantité thérapeutiquement efficace du composé, selon l'une quelconque des revendications 1 à 6, ou ses sels et promédicaments pharmaceutiquement acceptables.

7. Utilisation du composé, selon l'une quelconque des revendications 1 à 6, ou ses sels ou promédicaments pharmaceutiquement acceptables, pour fabriquer un médicament permettant de contrôler sélectivement la transmission synaptique par l'administration à un patient humain ou vétérinaire, ayant besoin d'un tel traitement, d'une quantité thérapeutiquement active dudit composé ou de son sel ou promédicament.

8. Utilisation d'un composé selon l'une quelconque des revendications 1 à 6, pour fabriquer un médicament pour traiter les démences, les troubles déficitaires de l'attention, l'anxiété associée à un dysfonctionnement cognitif ou à une suppression de la pharmacodépendance, **caractérisé par** une fonction cholinergique diminuée par l'administration à un patient humain ou vétérinaire, ayant besoin d'un tel traitement, d'une quantité thérapeutiquement efficace dudit composé.
